# EUROPEAN PATENT SPECIFICATION

(11) **EP 1 673 570 B1**
(45) Date of publication and mention of the grant of the patent: **05.03.2014**
(21) Application number: 04795367.4
(22) Date of filing: 14.10.2004
(51) Int. Cl.: F16L 33/00

(54) **AN IMPROVED BARB CLAMP**
VERBESSERTE HAKENKLEMME
BRIDE DE RACCORDEMENT RAINUREE AMELIOREE

(30) Priority: 17.10.2003 US 512231 P; 13.10.2004 US 963457
(43) Date of publication of application: 28.06.2006
(73) Proprietor: Saint-Gobain Performance Plastics Corporation, Aurora, OH 44202 (US)
(72) Inventor: WERTH, Albert, A., Kewadin, MI 49648 (US)
(74) Representative: Zimmermann & Partner
(86) International application number: PCT/US2004/034190
(87) International publication number: WO 2005/037343

(56) References cited:
- EP-A- 0 515 930
- EP-A- 0 762 034
- EP-A2- 0 823 578
- FR-A- 1 052 094
- US-A- 2 958 549
- US-A- 3 325 194
- US-A- 3 589 752
- US-A- 4 303 263
- US-A- 4 412 693
- US-A- 5 709 413
- US-A1- 2003 047 943
- US-A1- 2003 193 190
- US-B1- 6 435 568

## Description

### FIELD OF THE INVENTION

The present invention relates to a fastening device for a tubular body.

### BACKGROUND OF THE INVENTION

The transfer of fluid through flexible tubing is widely used in various environments. Ultimately, the flexible tubing is connected to the source of the gaseous or liquid fluid, the delivery site of the fluid, or to another flexible tubing. At the ends of the flexible tubing, it is necessary to provide a secure and leak proof connections. Although these requirements are necessary in all environments using flexible tubing, it is critical in the medical and pharmaceutical, food and beverage fields. In the medical and pharmaceutical fields flexible tubing and associated connections are used for luer fittings, quick connects, or sanitary fittings such as used in blood pumps, oxygen concentrators, sleep apnea equipment, medical transport containers, IV bags, etc. In some environments, and especially in the medical field, it is imperative to absolutely prohibit any liquids from getting between the tube and the connection fitting at the connection point. Any gap or abrupt change at the point of connection will entrap fluid, e. g. bio, blood, drugs, foodstuff, etc. This entrapment can cause growth of harmful bacteria.

### SUMMARY OF THE INVENTION

The present invention addresses the aforementioned concerns. The present invention is an improved barb clamp according to claim 1 used for joining a flexible tube to a barb fitting, or connector, or straight lumen without a barb. And, in particular, for joining flexible tube to a specially constructed barb fitting that provides minuscule transition differential between the inner diameter of the tubing and the inner diameter of the barb fitting.

The barb fitting tapers to a termination point so that there is essentially no transition between the inner diameter of the tube and the inner diameter of the bard fitting for preventing a pressure differential in the tube between a portion of the tube in the improved barb clamp and a portion of the tube outside of the barb clamp. Further features of the invention are described in the dependant claims.

The closest prior art US 2003/0193190 A1 describes a plastic barb clamp which provides leakproof connection of a flexible tube in a barb fitting. The barb clamp includes a collet and a sleeve. The barb clamp is configured to fit snugly within the tube. The collet is slid over the tube. Annular retainers on the interior surface of the collet fit tightly around the tube and under an expanded portion of the barb fitting. The sleeve is then slid over the collet. As the sleeve moves over the collet, tangs on the collet are pushed radially inwardly into the tube and barb fitting. The exterior surface of the collet has radial projections or expanded diameter portions for locking with the sleeve. The sleeve has an annular projection or a reduced diameter portion on the interior surface of the sleeve to correspond to the exterior surface of the collet to provide a secure lock between the collet and sleeve. To properly orientate the sleeve relative to the collet, the sleeve and collet can be molded as one piece, wherein the sleeve and collet are connected by thin, frangible pieces of plastic.

US 5709413 describes a fitting assembly for coupling tubing to a device having a sealing surface. The fitting includes a ferrule for seating against the sealing surface. The ferrule has a head. The ferrule is formed from a first material. A reusable collar having a bevelled rear surface and an inner surface is provided. The inner surface has a ring for gripping the tubing. The collar is formed of a second material that may differ from the first material and may be malleable. A coupler engages the head of the ferrule to push the ferrule against the sealing surface. The coupler has a ramped inner surface for engaging the rear surface to push the collar against the head of the ferrule. The coupler compresses the collar radially to grip the tubing.

Other applications of the present invention will become apparent to those skilled in the art when the following description of the best mode contemplated for practicing the invention is read in conjunction with the accompanying drawings.

### BRIEF DESCRIPTION OF THE DRAWING

The description herein makes reference to the accompanying drawings wherein like reference numerals refer to like parts throughout the several views, and wherein:
Figure 1 is a sectional view of a current barb clamp;
Figure 2 is a partial sectional view of an alternative to the current barb clamp;
Figure 3 is an elevational view of a collet for the current barb clamp;
Figure 4 is a sectional view of a sleeve for the current barb clamp;
Figure 5 is a sectional view of an improved barb clamp not according to the present invention; and
Figure 6 is a sectional view of an alternative embodiment of the improved barb clamp.

### DETAILED DESCRIPTION OF THE PREFERRED EMBODIMENT

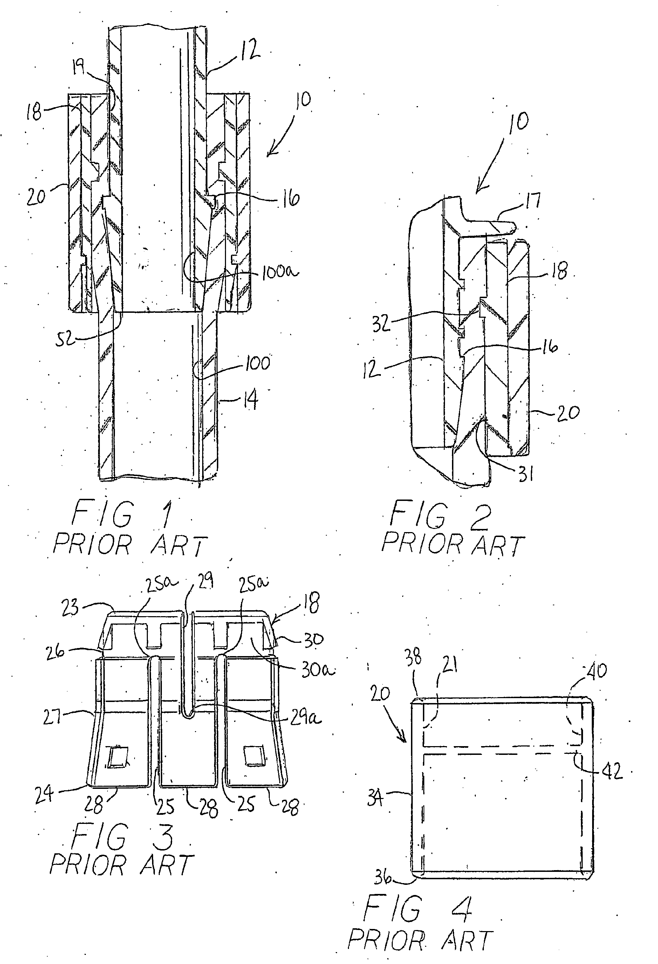

Figures 1 and 2 show the current barb clamp connector 10 for coupling a barbed fitting 12 and a flexible tube 14 and discussed hereafter. The barb fitting 12 is generally made of a non-metal material which allows it to be heat welded to a proprolyene or ethylene medical or pharmaceutical bag. The same and similar materials can be used for biotech, pharmaceutical, medical, and foodstuff fitting connections and manifold applications. The fitting can also be made from other plastics and stainless steel when required. As seen in Figures 1 and 2, the barbed fitting 12 may encompass different configurations but will generally include at least one expanded or barbed end 16 for a 360° radial compression connection to the flexible tube 14. If the barb clamp 10 is to be used in a medical or pharmaceutical environment, the barb fitting 12 is preferably made from an FDA (Food and Drug Administration) approved polypropylene, silicone, TPE, TPR, etc. The barb fitting 12 may also include a flanged portion 17 which defines a stop for the barb clamp 10.

The barb clamp 10 includes a collet 18 and a sleeve 20. The collet 18 and the sleeve 20 are as shown in Figures 1-6 and are similar to the collet and sleeve discussed in U. S. Patent Application Serial Number 10/100,519 filed on March 18, 2002, now U. S. Patent No. 6,796,586 issued on September 28, 2004, published as US 2003/0006610 A1 on January 9, 2003.

The collet 18 is an essentially annular member having a through aperture 19 for receiving the end of a tube 14 therein. The sleeve 20 is also an annular member with a through aperture 21 for receiving the end of the tube 14 as well as having a diameter for also receiving the collet 18 therein.

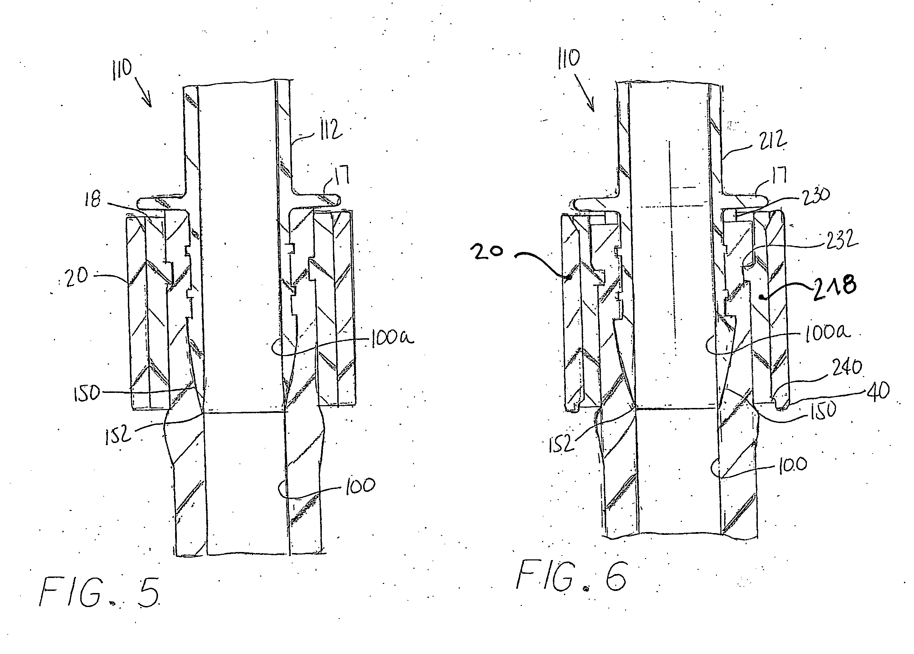

Looking at Figure 3 the collet 18 has an exterior surface 22 providing resilient means for radially contracting around the tube 14. The collet 18 has a first end 23 forming a discontinuous annular ring. Along the exterior surface 22 and adjacent to the first end 23 is an annular groove 26. Moving toward the second end 24 and beyond the annular groove 26, the collet forms eight resilient tangs 28. The tangs 28 radially flare out or expand slightly at the second end 24 of the collet 18 . The tangs 28 begin to flare approximately at the mid section 27 of each tang 28. The tangs 28 are formed by narrow through slots 25 extending from the second end 24 and terminating at the annular groove 26. The slots 25 are shown in Figures 3 and 5 with rounded termination ends 25a, however, the termination ends 25a may have pointed ends, (not shown).

A small ramping ledge 30 projects above each termination end 25a of the narrow through slots 25. The small ledges 30 provide added strength to the collet and also provide a stop means for the sleeve 20, as will be discussed hereinafter. Between each small ledge 30 there is a recessed planar portion 30a extending into the annular groove 26. The eight tangs 28 form a resilient seal which allow the tangs to contract around a tubular member 14. Between every other tang 28 there is a through slot 29 which extends from the first end 23 to the mid-section 27 of the associated tang 28. The through slots 29 may also have rounded termination ends 29a as shown in Figures 3 and 5 or pointed termination ends (not shown). The through slots 29 provide resiliency to the first end 23 of the collet 18 without sacrificing durability. The interior surface 31 of the collet 18 is essentially smooth except for a shelf 32 equally positioned on each tang 28 at the mid-section 27 for reasons to be discussed further.

Looking at Figure 4, the sleeve 20 has a smooth exterior annular surface 34. The sleeve 20 has a first or bottom end 36 forming an arcuate base to facilitate assembly to the collet 18. The interior surface 40 forms a slight outward taper at the second or top end 38 of the sleeve 20. The interior surface 40 is essentially smooth throughout the length of the sleeve 20 except for an annular projection 42 that extends from the inner surface. The annular projection 42 is sized and positioned on the sleeve 20 for disposition within the annular groove 26 of the collet 18 to form a lock when the barb clamp 10 is engaged. Therefore, the annular projection 42 is positioned proximate to the second or top end 38 of the sleeve 20.

The sleeve 20 is first placed over the end of the tube 14 so that the second or top end 38 of the sleeve 20 is spaced furthest away from the tube end The collet 18 is then placed on the tube 14 so that the first end 23 of the collet 18 is closest to the sleeve 20. The expanded end 16 of the barbed fitting 12 is then placed into the tube 14. The expanded end 16 of the barbed fitting 12 is sized for being snugly received within the interior of the tube 14. The collet 18 is then slid over the tube 14 having the expanded end 16 of the barbed fitting 12 therein. The shelves 32 located on the interior surface 31 1 of the collet 18 is a retainer which forms a radial 360° compression around the tube 14 and under the expanded end 16 of the barb fitting 12 so that the barb fitting 12 cannot easily move out of the tube 14. The sleeve 20 is then slid over the collet 18 such that the first or bottom end 36 of the sleeve 20 initially encounters the first end 23 of the collet 18. As the sleeve 20 moves over the collet 18, the tangs 28 on the collet 18 are pushed radially inwardly into the tube 14 and barbed fitting 12, so that the annular shelf 32 of the collet 18 is pressed inwardly into the tube 14 and barbed fitting 12 to provide a tight seal therebetween and thereby lock the annular shelf 32 under the barb 16. The sleeve 20 continues over the collet 18 until the annular projection 42 on the interior surface 40 of the sleeve 20 sits within the annular groove 26 of the collet 18. The small ledges 30 on the exterior surface 34 of the collet 18 provides a stop and lock to prevent the annular projection 42 from moving out of annular groove 26. The barb clamp 10 "clicks" when the collet 18 and sleeve 20 lock together. The barb clamp 10 can then only be removed with the aid of a tool so that disconnection and leakages are prevented.

The improved barb clamp shown in Figures 5 and 6 has many of the same features as discussed with regard to Figures 1-4. However, the improved barb clamp 10 further includes an engineered designed barb fitting 112 which is designed to maintain the same cross sectional area of the bore or fluid passageway 100a in the barb clamp 110 as the cross-sectional area of the bore or fluid passageway 100 in the tube 14 outside of the barb clamp 10. The fluid passageway 100 outside of the barb clamp 110 is defined by the inner bore of the tube 14. The fluid passageway 100a in the improved barb clamp 110 is defined by the inner bore of the barb fitting 112, 212. As can be seen in Figures 1 and 2, the cross-sectional area of the fluid passageway 100 of the tube 14 is reduced within the barb clamp 10 as shown at 100a in Figure 1. The free end 50 of the barb fitting 12 in the prior art has a blunt end 52 with a thickness that causes a transition in the cross-sectional area of the fluid passageway 100 as the tubing 14 enters the barb clamp 10 and changes to the fluid passageway 100a. One problem encountered when designing a barb connection is that it is generally necessary to have a smaller cross-sectional area of the bore in the barb fitting 12 in order to insert the barb fitting 12 into the tube 14. However, the improved clamp 10 provides a barb fitting 112, 212 that has essentially the same bore diameter as the tube 14, but can also easily be inserted into the tube 14.
In the improved barb clamp 110, as shown in Figures 5 and 6, the barb fitting 112, 212 has an engineered bore size that has the same cross-sectional area as the bore 100 of the tubing 14. When the improved barb fitting 112, 212 is installed into a tube 14, there is virtually no transition between the inner diameter of the tubing 14 and the inner diameter of the fitting 112. As a result, there is no pressure differential on the tubing or in the fluid from outside to inside of the barb clamp 110. Maintaining a constant pressure or a constant velocity of fluid throughout the system may be imperative in certain medical applications. To accomplish this, the free end 150 of the barb fitting 112, 212 is gradually tapered to a sharp point 152. The sharp point 152 on the free end 150 of the barb fitting 112 also allows for easy insertion of the barb fitting 112 into the tube 14. As a result of this configuration, liquid material can never get between the barb fitting 112, of 212, and the tube 14.

Figure 6 shows the inventive configuration of the barb clamp 110 which still maintains the same or equal inner diameter of the passageway of the tube 14 whether within the barb clamp or not. In Figure 6, it is shown that the barb fitting 212 includes a bumper stop 230 which is attached to the flange 17 to act as a separate stop for the tube 14 while the flange 17 defines another stop for the collet 218 and sleeve 20. The collet 218 may also be modified to have a tapered end 240 to correspond with a tapered end 40 of the sleeve. Further the annular shelf 232 of the collet 218 may be reconfigured to extend further into the tube 14 for a sturdier grip.

The collet 18 and sleeve 20 should be made of an FDA approved material if the barb clamp 10 is in a medical or pharmaceutical environment. According to the invention, the material of the collet and sleeve is resilient. Preferably the collet 18 is made of acetyl, silicone, or polypropylene. The sleeve 20 is preferably made of polycarbonate, silicone, or polypropylene. The components of the improved barb clamp are made of such material as polypropylene that can be sterilized in an autoclave for medical applications. Antimicrobial additives may also be added to the plastic material used in the manufacturing of at least one of the barb connector, collet, and sleeve.

While the invention has been described in connection with what is presently considered to be the most practical and preferred embodiment, it is to be understood that the invention is not to be limited to the disclosed embodiment but, on the contrary, is intended to cover various modifications and equivalent arrangements included within the scope of the appended claims.

## Claims

1. A barb clamp (110) for a flexible tube having a predetermined inner diameter essentially the same as an inner diameter of the barb clamp, the clamp (110) comprising a barb connector (212) and a collet (218) slidable over the first end of the barb connector (212), said collet having an external surface with an annular groove adjacent a first end and resilient tangs at a second end, wherein said resilient tangs flare outwardly along the external surface and are configured with an internal surface for, in use, contracting around the flexible tube, wherein:
the barb connector (212) has a tubular configuration with a first end (150) for disposing within an end of a flexible tube, said barb connector (212) having a smooth inner bore with essentially a constant inner diameter along an axial length thereof and at least one barb along an outer surface;
the barb clamp further comprising a cylindrical sleeve (20) having a through center aperture for receiving said collet (218), the sleeve (20) having an annular projection on its interior surface for reception in the annular groove; wherein
the cylindrical sleeve (20) and the collet (218) are made of a resilient material;
wherein the first end (150) of the barb connector (212) tapers to a termination point to provide, in use, essentially no transition from the inner diameter of the barb connector to essentially the same inner diameter of the flexible tube when the barb connector is assembled in the tube such that, in use, no pressure differential is created through the barb clamp; and
wherein the barb connector (212) includes a flange spaced from the first end (150) of the barb connector (212) and a bumper stop (230) attached to the flange (17), wherein the bumper stop (230) defines a stop for the tube (14) and the flange (17) defines a stop for the collet (218) and sleeve (20).

2. The barb clamp of claim 1 wherein the collet comprising an annular shelf (232), the cylindrical sleeve (20) slidable over the collet such that, in use, the tangs are pushed radially inwards so that the annular shelf (232) is locked under the barb of the barb connector (212).

3. The barb clamp (110) of claim 1 or claim 2, wherein the collet (18) and sleeve (20) have a locking ring configuration to, in use, prevent fluid material seeping between the barb connector (212) and the tube.

4. The barb clamp (110) of claim 1 or claim 2, wherein the collet (218) has a smooth interior surface with a radial shelf extending therein to, in use, form a radial 360° compression around the tube.

5. The barb clamp (110) of claim 1, wherein the sleeve (20) has a first end and the interior surface of the sleeve (20) has a slight outward taper at the first end.

6. The barb clamp (110) of claim 1 or claim 2, wherein said barb connector (212), collet (218), and sleeve (20) are made of a material capable of sterilization in an autoclave without damage.

7. The barb clamp (110) of claim 1 or claim 2, wherein at least one of the barb connector (212), collet (218) and sleeve (20) is made of a material having antimicrobial additives.

8. A tube assembly comprising a flexible tube and a barb clamp (110) according to claim 1 or claim 2, the tube having the same internal diameter as the internal diameter of the barb clamp.

## Patentansprüche

1. Hakenklemme (110) für einen flexiblen Schlauch mit einem vorab festgelegten Innendurchmesser, der im Wesentlichen gleich einem Innendurchmesser der Hakenklemme ist, wobei die Klemme (110) ein Hakenanschlussstück (212) und eine Spannzange (28) umfasst, die über das erste Ende des Hakenanschlussstücks (212) gleitbar ist, wobei die Spannzange eine Außenfläche mit einer ringförmigen Nut benachbart einem ersten Ende und flexible Zungen an einem zweiten Ende aufweist, wobei die flexiblen Zungen entlang der Außenfläche nach außen ausgeweitet und mit einer Innenfläche konfiguriert sind, um sich in Verwendung um den flexiblen Schlauch herum zusammen zu ziehen, wobei:
das Hakenanschlussstück (212) eine rohrförmige Konfiguration mit einem ersten Ende (150) aufweist, um innerhalb eines Endes eines flexiblen Schlauchs positioniert zu werden, wobei das Hakenanschlussstück (212) eine glatte Innenbohrung mit im Wesentlichen einem konstanten Innendurchmesser entlang einer Axiallänge davon und zumindest einen Haken entlang einer Außenfläche aufweist;
wobei die Hakenklemme darüber hinaus umfasst:
eine zylindrische Muffe (20) mit einer mittigen Durchgangsöffnung, um die Spannzange (28) aufzunehmen, wobei die Muffe (20) einen ringförmigen Vorsprung auf ihrer Innenfläche aufweist, um in der ringförmigen Nut aufgenommen zu werden; wobei
die zylindrische Muffe (20) und die Spannzange (28) aus einem biegsamen Material gebildet sind;
wobei sich das erste Ende (150) des Hakenanschlussstücks (212) in einen Endpunkt verjüngt, um in Verwendung im Wesentlichen keinen Übergang vom Innendurchmesser des Hakenanschlussstücks auf im Wesentlichen den gleichen Innendurchmesser des flexiblen Schlauches bereitzustellen, wenn das Hakenanschlussstück im Schlauch angeordnet ist, so dass in Verwendung keine Druckdifferenz durch die Hakenklemme gebildet wird; und
wobei das Hakenanschlussstück (212) einen von dem ersten Ende (150) des Hakenanschlussstücks (212) beabstandeten Flansch und einen an den Flansch (17) befestigten Pufferanschlag (230) enthält, wobei der Pufferanschlag (230) einen Anschlag für den Schlauch (14) definiert und der Flansch (17) einen Anschlag für die Spannzange (218) und die Muffe (20) definiert.

2. Hakenklemme nach Anspruch 1, wobei die Spannzange einen ringförmigen Vorsprung (232) umfasst, wobei die zylindrische Muffe (20) über die Spannzange gleitbar ist, so dass die Zungen in Verwendung radial einwärts gestoßen werden, so dass der ringförmige Vorsprung (232) unter dem Haken des Hakenanschlussstücks (212) verriegelt wird.

3. Hakenklemme (110) nach Anspruch 1 oder 2, wobei die Spannzange (18) und die Muffe (20) eine Sicherheitsringkonfiguration aufweisen, um in Verwendung zu verhindern, dass flüssiges Material zwischen dem Hakenanschlussstück (212) und dem Schlauch durchsickert.

4. Hakenklemme (110) nach Anspruch 1 oder 2, wobei die Spannzange (28) eine glatte Innenfläche mit einem radialen Vorsprung aufweist, der sich in diese erstreckt, um in Verwendung eine radiale 360°-Kompression um den Schlauch herum zu bilden.

5. Hakenklemme (110) nach Anspruch 1, wobei die Muffe (20) ein erstes Ende aufweist und die Innenfläche der Muffe (20) am ersten Ende eine geringfügige Verjüngung nach außen aufweist.

6. Hakenklemme (110) nach Anspruch 1 oder 2, wobei das Hakenanschlussstück (212), die Spannzange (28) und die Muffe (20) aus einem Material gebildet sind, das in einem Autoklaven ohne Beschädigung sterilisiert werden kann.

7. Hakenklemme (110) nach Anspruch 1 oder 2, wobei zumindest eines des Hakenanschlussstücks (212), der Spannzange (20) und der Muffe (20) aus einem Material mit antimikrobiellen Zusatzstoffen gebildet ist.

8. Schlauchanordnung, die einen flexiblen Schlauch und eine Hakenklemme (110) nach Anspruch 1 oder 2 umfasst, wobei der Schlauch den gleichen Innendurchmesser wie die Hakenklemme aufweist.

## Revendications

1. Bride de raccordement rainurée (110) pour un tube souple ayant un diamètre interne prédéterminé essentiellement identique à un diamètre interne de la bride de raccordement, la bride de raccordement (110) comprenant un raccord rainuré (212) et un collet (218) apte à coulisser sur la première extrémité du raccord rainuré (212), ledit collet ayant une surface externe présentant une gorge annulaire adjacente à une première extrémité et des tenons élastiques à une seconde extrémité, lesdits tenons élastiques s'évasant vers l'extérieur le long de la surface externe et étant configurés avec une surface interne pour, en utilisation, se contracter autour du tube souple, dans laquelle :
le raccord rainuré (212) a une configuration tubulaire avec une première extrémité (150) à disposer à l'intérieur d'une extrémité d'un tube souple, ledit raccord rainuré (212) ayant un alésage interne lisse ayant essentiellement un diamètre interne constant le long d'une longueur axiale de celui-ci et au moins une rainure le long d'une surface externe ;
la bride de raccordement rainurée comprenant en outre un manchon cylindrique (20) ayant une ouverture centrale traversante pour recevoir ledit collet (218), le manchon (20) ayant une saillie annulaire sur sa surface intérieure pour la réception dans la rainure annulaire ;
le manchon cylindrique (20) et le collet (218) étant faits d'une matière élastique ;
la première extrémité (150) du raccord rainuré (212) s'effilant jusqu'à un point de fin pour ne créer, en utilisation, essentiellement aucune transition du diamètre interne du raccord rainuré à essentiellement le même diamètre interne du tube souple lorsque le raccord rainuré est assemblé dans le tube de telle sorte que, en utilisation, aucun différentiel de pression n'est créé à travers la bride de raccordement ; et
le raccord rainuré (212) comprend une collerette espacée de la première extrémité (150) du raccord rainuré (212) et une butée d'arrêt (230) fixée à la collerette (17), la butée d'arrêt (230) définissant une butée pour le tube (14) et la collerette (17) définissant une butée pour le collet (218) et le manchon (20).

2. Bride de raccordement rainurée selon la revendication 1, dans laquelle le collet comprend un épaulement annulaire (232), le manchon cylindrique (20) étant apte à coulisser sur le collet de telle sorte qu'en utilisation les tenons sont poussés radialement vers l'intérieur de telle sorte que l'épaulement annulaire (232) est bloqué sous la rainure du raccord rainuré (212).

3. Bride de raccordement rainurée (110) selon l'une des revendications 1 ou 2, dans laquelle le collet (218) et le manchon (20) ont une configuration de bague de verrouillage pour, en utilisation, empêcher l'infiltration de matière fluide entre le raccord rainuré (212) et le tube.

4. Bride de raccordement rainurée (110) selon l'une des revendications 1 ou 2, dans laquelle le collet (218) a une surface intérieure lisse avec un épaulement radial s'étendant dans celui-ci pour, en utilisation, former une compression radiale à 360° autour du tube.

5. Bride de raccordement rainurée (110) selon la revendication 1, dans laquelle le manchon (20) a une première extrémité et la surface intérieure du manchon (20) a un léger évasement à la première extrémité.

6. Bride de raccordement rainurée (110) selon l'une des revendications 1 ou 2, dans laquelle lesdits raccord rainuré (212), collet (218) et manchon (20) sont faits d'une matière capable de stérilisation dans un autoclave sans endommagement.

7. Bride de raccordement rainurée (110) selon l'une des revendications 1 ou 2, dans laquelle au moins l'un du raccord rainuré (212), du collet (218) et du manchon (20) est fait d'une matière ayant des additifs antimicrobiens.

8. Ensemble tube comprenant un tube souple et une bride de raccordement rainurée (110) selon l'une des revendications 1 ou 2, le tube ayant le même diamètre interne que le diamètre interne de la bride de raccordement rainurée.
